# EUROPÄISCHE PATENTANMELDUNG

(11) **EP 2 363 089 A2**
(43) Veröffentlichungstag der Anmeldung: **07.09.2011**
(21) Anmeldenummer: 11001751.4
(22) Anmeldetag: 03.03.2011
(51) Int. Cl.: A61B 19/00, A61F 9/007

(54) **Medizinisches Handgerät, Handstück, Handgriff, etc.**

(30) Priorität: 03.03.2010 DE 102010010162; 08.06.2010 DE 102010023002
(71) Anmelder: Geuder AG, 69126 Heidelberg (DE)
(72) Erfinder: Geuder, Volker, 69126 Heidelberg (DE); Frauenfeld, Dieter, 69121 Heidelberg (DE); Wolschendorf, Marc, 69121 Heidelberg (DE)
(74) Vertreter: Ullrich & Naumann

(57) **Zusammenfassung**

Ein medizinisches Handgerät, Handstück, Handgriff, Schlauch, Lichtleiter oder sonstiges adaptierbares Zubehör zum Anschluss an eine Steuer-/Versorgungseinheit (3) zum Betreiben des Handgeräts (1), insbesondere chirurgisches Handgerät zur Durchführung ophthalmologischer Operationen, wobei das Handgerät (1) Kopplungsmittel zum Anschluss an eine sich zwischen der Steuer-/Versorgungseinheit (3) und dem Handgerät (1) erstreckenden Versorgungs-/Entsorgungsleitung (2) aufweist, ist dadurch gekennzeichnet, dass dem Handgerät (1), dem Handstück, dem Handgriff, dem Schlauch, dem Lichtleiter, etc. oder deren Verpackung Identifizierungsmittel (4) mit einem Identifizierungsmerkmal zugeordnet sind, die durch die Steuer-/Versorgungseinheit (3) lesbar bzw. erkennbar sind und dass nach dem Lesen bzw. Erkennen des Identifizierungsmerkmals ein ordnungsgemäßer Betrieb des Handgeräts (1) etc. insbesondere unter Nutzung aller Funktionen und Leistungsstufen, durch eine handgerätspezifische Aktivierung/Freischaltung der Steuer-/Versorgungseinheit (3) möglich ist.

## Beschreibung

Die vorliegende Erfindung betrifft ein medizinisches Handgerät, Handstück, Handgriff, Schlauch, Lichtleiter oder sonstiges adaptierbares Zubehör zum Anschluss an eine Steuer-/Versorgungseinheit zum Betreiben des Handgeräts, insbesondere chirurgisches Handgerät zur Durchführung ophthalmologischer Operationen, wobei das Handgerät Kopplungsmittel zum Anschluss an eine sich zwischen der Steuer-Nersorgungseinheit und dem Handgerät erstreckenden Versorgungs-/Entsorgungsleitung aufweist.

Medizinische Handgeräte der gattungsbildenden Art sind in den unterschiedlichsten Ausführungen aus der Praxis bekannt. So kann es sich dabei beispielsweise um ein chirurgisches Instrument zur Operation am menschlichen Auge handeln, beispielsweise um ein chirurgisches Instrument, wie es aus der EP 1 844 705 B1 bekannt ist. Dort geht es im Konkreten um ein zur Beleuchtung des Inneren des Auges dienendes Instrument, welches von einer Steuer-/Versorgungseinheit her über einen Lichtleiter als Versorgungsleitung mit Licht versorgt wird.

Des Weiteren kann es sich bei dem hier in Rede stehenden Handgerät um ein augenchirurgisches Instrument zum Zertrümmern von Linsen und zum Absaugen von Linsentrümmern handeln, wie es aus der DE 40 08 594 C2 bekannt ist. In diesem Falle wird das Handgerät von der Steuer-/Versorgungseinheit her mit pulsierender Druckluft und Spülflüssigkeit versorgt, wobei die zertrümmerten Linsenteile per Unterdruck abgesaugt werden. Hier dient die Schlauchverbindung zwischen dem Handgerät und der Steuer-/Versorgungseinheit sowohl zur Versorgung als auch zur Entsorgung.

An dieser Stelle sei angemerkt, das es für die erfindungsgemäße Lehre keine Rolle spielt, um welche konkreten medizinischen Handgeräte, Handstücke, Handgriffe, Schläuche, Lichtleiter es sich handeln mag. Letztendlich geht es hier nicht nur um medizinische Handgeräte oder dergleichen, vielmehr auch um adaptierbares Zubehör, welches zum unmittelbaren oder mittelbaren Anschluss an eine Steuer-/Versorgungseinheit verwendet wird. Dabei ist wesentlich, dass das Handgerät, möglicherweise unter Zwischenschaltung des adaptierbaren Zubehörs, über eine Steuer-/Versorgungseinheit gesteuert und versorgt sowie entsorgt wird, wobei als Wirkverbindung eine Versorgungs-/Entsorgungsleitung zwischen der Steuer/Versorgungseinheit und dem Handgerät vorgesehen ist. Dies kann auch einen elektrischen Stromanschluss umfassen.

In der Praxis kommt es häufig vor, dass beispielsweise ein auf dem Gebiet der Ophthalmologie tätiger Operateur eine Steuer-Nersorgungseinheit eines konkreten Herstellers nutzt, jedoch Geräte, Handstücke oder Handgriffe anderer Hersteller anschließt, die sich zwar in einem gewissen Umfange von der Steuer-/Versorgungseinheit steuern, regeln und wie auch immer beaufschlagen, versorgen und entsorgen lassen, jedoch mit hohen Risiken im Betrieb, nämlich in Ermangelung einer 100%igen Adaption. Sofern es während des Betriebs zu einem Versagen des Handgeräts kommt, sind Unfälle in Verlaufe des operativen Eingriffs nicht auszuschließen.

In der Praxis gibt es bereits eine - mehr oder weniger praktikable - Lösung des voranstehenden Problems, nämlich dahingehend, dass die Handgeräte, Handstücke, Handgriffe, etc. mit einer Art mechanischer Codierung versehen sind, so dass an die geräteseitig gelieferte Versorgungs-/Entsorgungsleitung nur solche Handgeräte ankoppelbar sind, die die passende mechanische Codierung zum Andocken bzw. Ankoppeln aufweisen. Durch Nachahmung entsprechender Anschlussstücke kann eine solche Codierung umgangen werden. Die bekannte Problematik bleibt erhalten.

Der vorliegenden Erfindung liegt daher die Aufgabe zugrunde, ein medizinisches Handgerät, ein Handstück, einen Handgriff, einen Schlauch, einen Lichtleiter oder sonstiges adaptierbares Zubehör zum Anschluss an eine Steuer-Nersorgungseinheit zum Betreiben des Handgeräts, insbesondere ein chirurgisches Handgerät zur Durchführung ophthalmologischer Operationen, anzugeben, wonach gewährleistet ist, dass es ausschließlich an die geeigneten bzw. dazu passende Steuer-Nersorgungseinheit anschließbar ist. Mit anderen Worten ist sicherzustellen, dass an eine vorgegebene Steuer-Nersorgungseinheit nur die dafür konzipierten Handgeräte bzw. das dafür konzipierte Zubehör ankoppelbar und - vollumfänglich - betreibbar ist.

Die voranstehende Aufgabe ist durch die Merkmale des Patentanspruchs 1 gelöst. Danach ist das erfindungsgemäße medizinische Handgerät dadurch gekennzeichnet, dass dem Handgerät, dem Handstück, dem Handgriff, dem Schlauch, dem Lichtleiter, dem adaptierbaren Zubehör oder deren Verpackung Identifizierungsmittel mit einem Identifizierungsmerkmal zugeordnet sind, die durch die Steuer-/Versorgungseinheit lesbar bzw. erkennbar sind und dass nach dem Lesen bzw. Erkennen des Identifizierungsmerkmals ein ordnungsgemäßer Betrieb des Handgeräts etc. insbesondere unter Nutzung aller Funktionen und Leistungsstufen, durch eine handgerätspezifische Aktivierung/Freischaltung der Steuer-/Versorgungseinheit möglich ist.

Erfindungsgemäß ist erkannt worden, dass es möglich ist, dem Handgerät bzw. dem Handstück oder dem Handgriff, dem Schlauch, dem Lichtleiter oder dem sonstigen adaptierbaren Zubehör oder der Verpackung des Handgeräts, etc. Identifizierungsmittel zuzuordnen, die ein möglichst eindeutiges Identifizierungsmerkmal beinhalten bzw. generieren. Das Identifizierungsmerkmal ist durch die Steuer-/Versorgungseinheit lesbar bzw. durch diese erkennbar, wobei erst nach dem Lesen bzw. Erkennen des Identifizierungsmerkmals ein ordnungsgemäßer Betrieb des Handgeräts möglich ist, wenn nämlich das Identifizierungsmerkmal den Vorgaben in der Steuer-/Versorgungseinheit entspricht. Nur dann lassen sich alle Funktionen und Leistungsstufen nutzen, nämlich durch eine handgerätespezifische Aktivierung/Freischaltung der Steuer-/Versorgungseinheit. Ein Missbrauch, insbesondere durch unsachgerechte Nutzung nicht passender Handgeräte, Handstücke oder Handgriffe in Verbindung mit einer beliebigen Steuer-/Versorgungseinheit ist insoweit ausgeschlossen.

In vorteilhafter Weise sind in der Steuer-/Versorgungseinheit relevanten Identifizierungsmerkmale zum Abgleich abgelegt, die mit einem jeweiligen Identifizierungsmerkmal des Handgeräts übereinstimmen müssen.

Im Rahmen einer einfachen Ausgestaltung der erfindungsgemäßen Lehre ist es möglich, dass das Identifizierungsmerkmal durch ein vorzugsweise der Steuer-/Versorgungseinheit zugeordnetes Lesegerät insbesondere lichtoptisch erkennbar und in die Steuer-/Versorgungseinheit einlesbar ist. Bei dem Identifizierungsmerkmal kann es sich beispielsweise um einen Barcode oder dgl. handeln, der beim Erwerb des Handgeräts erhältlich oder aufgrund eines Kaufnachweises über das Internet abrufbar und der Steuer-/Versorgungseinheit zuführbar ist. Eine Online-Verbindung der Steuer-Nersorgungseinheit, beispielsweise über das Internet, mit dem Hersteller der Steuer-/Versorgungseinheit und der Handgeräte ist denkbar, so dass bereits mit dem Abverkauf eines konkreten Handgeräts bzw. der Auslieferung des Handgeräts die Steuer-/Versorgungseinheit - für den Kunden quasi automatisch - mit entsprechenden Identifizierungsmerkmalen ausgestattet wird.

In besonders vorteilhafter und dabei raffinierter Weise sind die Identifizierungsmittel, die letztendlich das Identifizierungsmerkmal generieren oder beinhalten, in dem Handgerät, vorzugsweise im Handgriff des Handgeräts, vorgesehen. Im Konkreten können die Identifizierungsmittel in einem endseitigen Handgerät-Stecker vorgesehen sein, an den die Versorgungs-/Entsorgungsleitung, zumindest aber eine Stromleitung, mit einer entsprechenden Buchse angeschlossen wird. Ebenso ist es denkbar, dass die Identifizierungsmittel sowohl im Handgerät bzw. in dem endseitigen Handgerät-Stecker als auch in einer Anschlussbuchse der Versorgungs-/Entsorgungsleitung bzw. einer elektrischen Leitung oder in einer Buchse in der Steuer-Nersorgungseinheit vorgesehen sind, wobei sich die Identifizierungsmittel beider Bauteile zur Definition des Identifizierungsmerkmals ergänzen. Auch durch diese Maßnahme ist eine weitere Stufe an Sicherheit realisiert.

Im Konkreten ist es von Vorteil, dass im Handgriff bzw. im Handgriff-Stecker ein elektronisches Bauteil, vorzugsweise ein Widerstand, angeordnet ist, das nach Ankopplung an die Versorgungs-/Entsorgungsleitung über eine Konstantstromquelle gespeist und über eine elektrische Schaltung, beispielsweise als Element einer Brückenschaltung oder dgl., von der Steuer-Nersorgungseinheit detektierbar bzw. erkennbar ist. So könnte im Konkreten der Wert des jeweiligen Widerstands zu einem Brückenabgleich führen. Wird ein Handgerät ohne elektrischen Widerstand oder mit einem "falschen" Widerstand angeschlossen, findet der Brückenabgleich nicht statt, so dass aufgrund einer entsprechenden Detektion eine Freischaltung des Handgeräts über die Steuer-Nersorgungseinheit verweigert wird. Ebenso könnte einfach der Widerstand ermittelt und verglichen werden.

Die Stromversorgung und ggf. weitere elektronische Bauteile, abseits des Handgeräts, sind in weiter vorteilhafter Weise in die Steuer-/Versorgungseinheit integriert, wobei eine Integration auch in die Versorgungs-/Entsorgungsleitung, beispielsweise in deren Anschlüsse oder in einer separate Stromleitung bzw. dessen Stecker, vorgesehen sein kann, so dass ein Abgleich zwischen dem Handgerät, der Versorgungs-/Entsorgungsleitung und der Steuer-/Versorgungseinheit in Bezug auf den Hersteller möglich ist. Dies ist von weiterem Vorteil.

Des Weiteren kann ein Mikrocontroller zur Signalverarbeitung und Weiterleitung der ausgewerteten Signale, insbesondere zur Generierung eines Freigabesignals in der Steuer-/Versorgungseinheit, vorgesehen sein. Die Integration einer geeigneten Prozessortechnik, beispielsweise als Bestandteil eines in der Steuer-/Versorgungseinheit ohnehin vorgesehen Prozessors, ist denkbar.

Wie bereits zuvor erwähnt, ist es grundsätzlich denkbar, dass die Steuer-/Versorgungseinheit in Ermangelung eines passenden Identifizierungsmerkmals am bzw. im Handgerät oder bei Detektion eines in der Steuer-/Versorgungseinheit nicht hinterlegten Identifizierungsmerkmals das Handgerät nicht unterstützt. Dies kann bedeutet, dass dem Handgerät jedwede Unterstützung bzw. Versorgung/Entsorgung verweigert wird.

Alternativ ist es denkbar, das die Steuer-/Versorgungseinheit in Ermangelung eines passenden Identifizierungsmerkmals am bzw. im Handgerät oder bei Detektion eines in der Steuer-Nersorgungseinheit nicht hinterlegten Identifizierungsmerkmals das Handgerät zwar aktiviert, jedoch nicht alle Funktionen unterstützt. Auch ist es möglich, dass die Funktionen, beispielsweise betreffend Lichtstärke, Luftdruck, Unterdruck, etc. mit nur reduzierter Leistung aktiviert bzw. unterstützt werden. Eine Art Notbetrieb wäre dann möglich, wobei zur Ausnutzung der gesamten Leistungsfähigkeit der Steuer-Nersorgungseinheit unter Nutzung des Handgeräts ein passendes Handgerät erforderlich ist.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht die prinzipielle Anordnung von SteuerNersorgungseinheit, Handgerät und verbindender Versorgungs-/Entsorgungsleitung und
- Fig. 2: in einer schematischen Ansicht im Sinne eines Blockschaltbildes, das Zusammenwirken von Handgerät und Identifizierungsmitteln, die im Handgerät und in der Steuer-/Versorgungseinheit untergebracht sind.

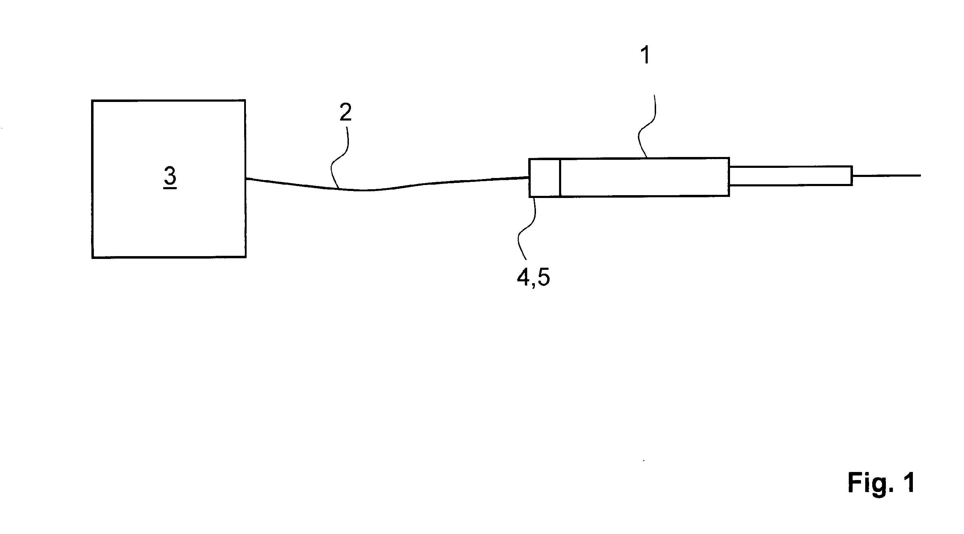

Fig. 1 zeigt in einer vereinfachten schematischen Ansicht Darstellung ein medizinisches Handgerät 1, welches mit einer Steuer-/Versorgungseinheit 3 verbunden ist. Die zuvor genannten Komponenten und Einheiten sind als autarkes System zu verstehen, welches von Außen her lediglich eine Stromversorgung benötigt.

Erfindungsgemäß sind dem Handgerät 1 Identifizierungsmittel 4 zur Ausgabe eines Identifizierungsmerkmals zugeordnet.

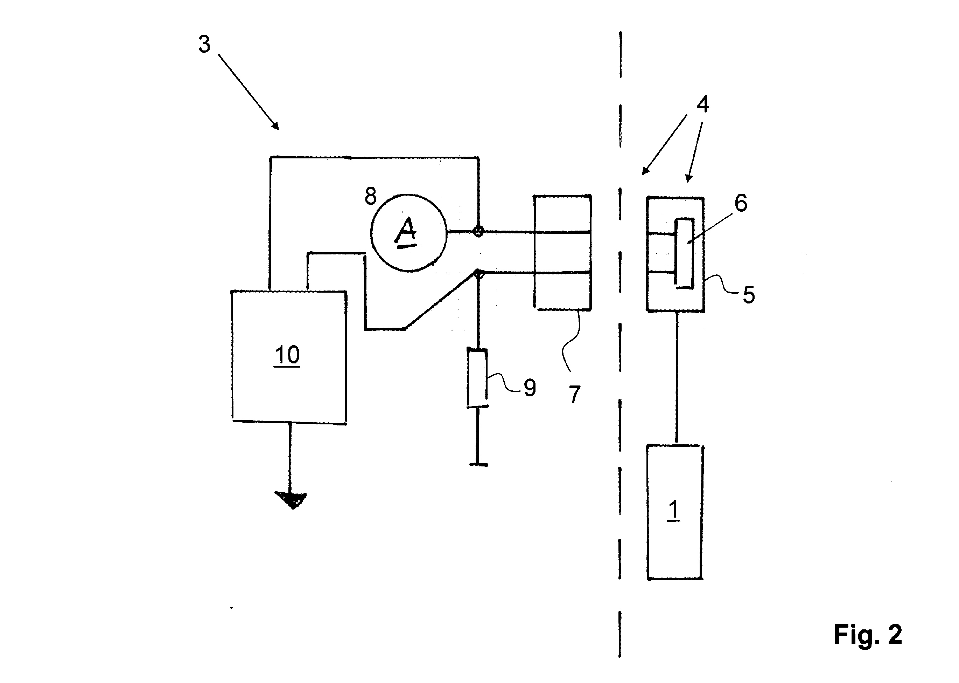

Bei dem in Fig. 2 gezeigten Ausführungsbeispiel umfasst das Handgerät 1 bzw. dessen Handgriff einen Handgriff-Stecker 5, in den ein elektrischer Widerstand 6 integriert ist. Das Handgerät 1 ist durch einen Handgriff-Stecker 5 über die in Fig. 2 nicht gezeigte Versorgungs-/Entsorgungsleitung mit einer Buchse 7 in der Steuer-/Versorgungseinheit 3 verbunden, wobei dazu - abseits der Versorgungs-/Entsorgungsleitung - ein elektrisches Kabel dienen kann, welches sich zwischen dem Handgriff-Stecker 6 und der Buchse 7 erstreckt. Eine Kombination mit der Versorgungs-/Entsorgungsleitung 2 ist möglich.

Von der Steuer-/Versorgungseinheit 3 aus wird die zur Generierung des Identifizierungsmerkmals dienende elektrische Schaltung mit Konstantstrom versorgt, nämlich über eine Konstantstromquelle 8. Der elektrische Widerstand 6 kann wie auch ein weiterer, zur elektrischen Schaltung gehörender Widerstand 9 Teil einer Brückenschaltung sein, wobei bei entsprechender Ankopplung eines geeigneten elektrischen Widerstands 6 im Handgriff-Stecker 5 ein Brückenabgleich erfolgt. Auch ist es denkbar, dass lediglich der elektrische Widerstand 6 im Handgriff-Stecker 5 - als solcher - detektiert wird. Beliebige Auslegungen der elektrischen/ elektronischen Schaltung sind denkbar.

Zur Signalaufbereitung und Verarbeitung dient ein Mikrocontroller 10, so dass von dort aus eine Aktivierung der Steuer-/Versorgungseinheit 3 erfolgt.

Hinsichtlich weiterer Merkmale, die sich den beiden Fig. nicht entnehmen lassen, sei zur Vermeidung von Wiederholungen auf den allgemeinen Teil der Beschreibung verwiesen.

Schließlich sei angemerkt, dass das voranstehend erörterte Ausführungsbeispiel lediglich der beispielhaften Erörterung der beanspruchten Lehre dient, diese jedoch nicht auf die Ausführungsbeispiel einschränkt.

### Bezugszeichenliste

- 1: medizinisches Handgerät, Handgriff
- 2: Versorgungs-/Entsorgungsleitung
- 3: Steuer-/Versorgungseinheit
- 4: Identifizierungsmittel
- 5: Handgriff-Stecker
- 6: elektrischer Widerstand (im Handgriff-Stecker)
- 7: Buchse
- 8: Gleichstromquelle
- 9: elektrischer Widerstand (in der Steuer-/Versorgungseinheit)
- 10: Mikrocontroller

## Patentansprüche

1. Medizinisches Handgerät, Handstück, Handgriff, Schlauch, Lichtleiter oder sonstiges adaptierbares Zubehör zum Anschluss an eine Steuer-/Versorgungseinheit (3) zum Betreiben des Handgeräts (1), insbesondere chirurgisches Handgerät zur Durchführung ophthalmologischer Operationen, wobei das Handgerät (1) Kopplungsmittel zum Anschluss an eine sich zwischen der Steuer-/Versorgungseinheit (3) und dem Handgerät (1) erstreckenden Versorgungs-/Entsorgungsleitung (2) aufweist,
**dadurch gekennzeichnet, dass** dem Handgerät (1), dem Handstück, dem Handgriff, dem Schlauch, dem Lichtleiter, etc. oder deren Verpackung Identifizierungsmittel (4) mit einem Identifizierungsmerkmal zugeordnet sind, die durch die Steuer-/Versorgungseinheit (3) lesbar bzw. erkennbar sind und dass nach dem Lesen bzw. Erkennen des Identifizierungsmerkmals ein ordnungsgemäßer Betrieb des Handgeräts (1) etc. insbesondere unter Nutzung aller Funktionen und Leistungsstufen, durch eine handgerätspezifische Aktivierung/Freischaltung der Steuer-/Versorgungseinheit (3) möglich ist.

2. Handgerät nach Anspruch 1, **dadurch gekennzeichnet, dass** in der Steuer-/Versorgungseinheit (3) relevante Identifizierungsmerkmale zum Abgleich abgelegt sind.

3. Handgerät nach Anspruch 1 oder 2, **dadurch gekennzeichnet, dass** das Identifizierungsmerkmal durch ein vorzugsweise der Steuer-/Versorgungseinheit (3) zugeordnetes Lesegerät vorzugsweise lichtoptisch, erkennbar und in die Steuer-/Versorgungseinheit (3) einlesbar ist.

4. Handgerät nach einem der Ansprüche 1 bis 3, **dadurch gekennzeichnet, dass** als Identifizierungsmerkmal eine Barcode oder dgl. dient, der beim Erwerb des Handgeräts (1) erhältlich oder aufgrund eines Kaufnachweises über das Internet abrufbar und der Steuer-/Versorgungseinheit (3) zuführbar ist.

5. Handgerät nach Anspruch 1 oder 2, **dadurch gekennzeichnet, dass** die Identifizierungsmittel (4) in dem Handgerät (1), vorzugsweise im Handgriff, vorgesehen sind.

6. Handgerät nach Anspruch 5, **dadurch gekennzeichnet, dass** die Identifizierungsmittel (4) in einem endseitigen Handgerät-Stecker (5) vorgesehen sind.

7. Handgerät nach Anspruch 5 oder 6, **dadurch gekennzeichnet, dass** die Identifizierungsmittel (4) sowohl im Handgerät (1) bzw. in dem endseitigen Handgerät-Stecker (5) als auch in einer Anschlussbuchse der Versorgungs-/Entsorgungsleitung (2) vorgesehen sind, wobei sich die Identifizierungsmittel (4) beider Bauteile zur Definition des Identifizierungsmerkmals ergänzen.

8. Handgerät nach einem der Ansprüche 5 bis 7, **dadurch gekennzeichnet, dass** im Handgriff (1) bzw. im Handgriff-Stecker (5) ein elektronisches Bauteil (6), vorzugsweise ein Widerstand, angeordnet ist, das nach Ankopplung an die Versorgungs-/Entsorgungsleitung (2) über eine Gleichspannungsquelle gespeist und über eine elektrische Schaltung, beispielsweise als Element einer Brückenschaltung oder dgl., von der Steuer-/Versorgungseinheit detektierbar bzw. erkennbar ist.

9. Handgerät nach Anspruch 8, **dadurch gekennzeichnet, dass** die Stromversorgung und ggf. weitere elektronische Bauteile (9) in die Steuer-/Versorgungseinheit integriert sind.

10. Handgerät nach Anspruch 8 oder 9, **dadurch gekennzeichnet, dass** ein Mikrocontroller (10) zur Signalverarbeitung und Weiterleitung der ausgewerteten Signale dient.

11. Handgerät nach einem der Ansprüche 1 bis 10, **dadurch gekennzeichnet, dass** die Steuer-/Versorgungseinheit (3) in Ermangelung eines passenden Identifizierungsmerkmals am bzw. im Handgerät (1) oder bei Detektion eines in der Steuer-Nersorgungseinheit (3) nicht hinterlegten Identifizierungsmerkmals das Handgerät (1) nicht unterstützt.

12. Handgerät nach einem der Ansprüche 1 bis 10, **dadurch gekennzeichnet, dass** die Steuer-/Versorgungseinheit (3) in Ermangelung eines passenden Identifizierungsmerkmals am bzw. im Handgerät (1) oder bei Detektion eines in der Steuer-/Versorgungseinheit (3) nicht hinterlegten Identifizierungsmerkmals nicht alle Funktionen oder Funktionen mit nur reduzierter Leistung aktiviert bzw. unterstützt.

13. Handgerät nach einem der Ansprüche 1 bis 12, **dadurch gekennzeichnet, dass** aufgrund eines erkannten Identifizierungsmerkmals die Steuer-/Versorgungseinheit (2) automatisch auf die vom konkreten Handgerät (1) abrufbaren bzw. aktivierbaren Funktionen und Leistungen eingestellt wird.
